# EUROPEAN PATENT SPECIFICATION

(11) **EP 4 176 807 B1**
(45) Date of publication and mention of the grant of the patent: **05.06.2024**
(21) Application number: 21206162.6
(22) Date of filing: 03.11.2021
(51) Int. Cl.: A61B 5/08, A61B 5/113, A61B 5/18

(54) **A SYSTEM FOR DEFINING A STRESS LEVEL OF A PERSON WITHIN A VEHICLE**
SYSTEM ZUR DEFINIERUNG EINES STRESSNIVEAUS EINER PERSON IN EINEM FAHRZEUG
SYSTÈME PERMETTANT DE DÉFINIR UN NIVEAU DE CONTRAINTE D'UNE PERSONNE À L'INTÉRIEUR D'UN VÉHICULE

(43) Date of publication of application: 10.05.2023
(73) Proprietor: Valeo Comfort and Driving Assistance, 94000 Créteil (FR)
(72) Inventor: BLIEN, Thilo, 94000 Créteil (FR); KAISER, Frank, 94000 Créteil (FR); ANIL, Anand, 94000 Créteil (FR); DOENTGEN, Bernhard, 94000 Créteil (FR); KIEFER, Tobias, 94000 Créteil (FR)
(74) Representative: Delplanque, Arnaud

(56) References cited:
- US-A1- 2012 116 202
- US-A1- 2019 328 278
- SHAN YUHAO ET AL: "Respiratory signal and human stress: non-contact detection of stress with a low-cost depth sensing camera", INTERNATIONAL JOURNAL OF MACHINE LEARNING AND CYBERNETICS, SPRINGER BERLIN HEIDELBERG, BERLIN/HEIDELBERG, vol. 11, no. 8, 24 February 2020 (2020-02-24), pages 1825-1837, XP037191384, ISSN: 1868-8071, DOI: 10.1007/S13042-020-01074-X [retrieved on 2020-02-24]

## Description

The present invention relates to a system for defining a stress level of an occupant within a cabin of a vehicle. Such a system may be used, but not exclusively, in a vehicle.

Different systems for monitoring breathing of a person are well-known by the man skilled in the art. Such systems for monitoring are used to assess the fitness of an athlete in the field of sport, the sleeping behavior of a person in the medical field or to identify breathing anomalies in the medical field.

One problem of these prior arts is that they do not assess the problem of stress level. Now, in the automotive field, there is a need to check the stress level of a person of a vehicle, in particular the driver of the vehicle, such as a motor vehicle, in order to ensure the safety of all the occupants of said vehicle. US 2019/328278 A1 and SHAN YUHAO ET AL: "Respiratory signal and human stress: non-contact detection of stress with a low-cost depth sensing camera", INTERNATIONAL JOURNAL OF MACHINE LEARNING AND CYBERNETICS, SPRINGER BERLIN HEIDELBERG, BERLIN/ HEIDELBERG, vol. 11, no. 8, 24 February 2020 (2020-02-24), pages 1825-1837, ISSN: 1868-8071, DOI: 10.1007/S13042-020-01074-X relate to determining a stress level. US 2012/116202 A1 relates to determining a best feature subset for distinguishing and recognizing stress.

It is an object of the invention to provide a system for defining a stress level of a person within a cabin of a vehicle, which resolves the problem above-stated.

To this end, it is provided a system for defining a stress level of a person within a cabin of a vehicle, wherein said system comprises:
- at least one sensor configured to measure:
   o an upper body movement of a person,
   o a current breathing amplitude based on said upper body movement,
   o an inhaling duration and an exhaling duration of the breathing of said person based on said upper body movement,
   o a current breathing rate based on said inhaling duration and said exhaling duration,
- an electronic control unit configured to:
   o compare the inhaling duration to the exhaling duration,
   o compare said current breathing rate to a mean breathing rate, and based on said comparison, characterize the current breathing rate as slow, average or fast,
   o compute a maximum breathing amplitude based on a maximum breathing depth,
   o compare said maximum breathing amplitude to a mean breathing amplitude, and based on said comparison, characterize the breathing amplitude as flat, average, or deep,
   o based on the characterization of the breathing amplitude, on the characterization of the current breathing rate and on the comparison of the inhaling duration to the exhaling duration, determine the stress level of said person within the cabin.

According to non-limitative embodiments of the invention, the system in accordance with the invention further comprises the following characteristics.

In a non-limitative embodiment, said sensor is a radar sensor or an optical sensor.

In a non-limitative embodiment, the electronic control unit is further configured to detect a yawning based on said upper body movement, said yawning defining the maximum breathing depth.

In a non-limitative embodiment, the measurement of the current breathing rate is a long term measurement performed during a journey of said vehicle.

In a non-limitative embodiment, the stress level is defined as relaxed, slightly stressed, and stressed.

In a non-limitative embodiment, the stress level is relaxed when:
- the current breathing rate is slow, and
- the breathing amplitude is deep, and
- the inhaling duration is shorter than the exhaling duration

In a non-limitative embodiment, the stress level is slightly stressed when:
- the current breathing rate is average, and
- the breathing amplitude is equal to the mean breathing amplitude, and
- the inhaling duration is equal to the exhaling duration.

In a non-limitative embodiment, the stress level is stressed when:
- the current breathing rate is fast, and
- the breathing amplitude is flat, and
- the inhaling duration is longer than the exhaling duration.

In a non-limitative embodiment, the stress level comprises a plurality of sub-levels.

In a non-limitative embodiment, the mean breathing amplitude is computed from a plurality of measures of the current breathing amplitude.

There is also provided a method for defining a stress level of a person within a cabin of a vehicle, wherein said method comprising:
- measurement by at least one sensor of an upper body movement of a person,
- measurement by said at least one sensor of a current breathing amplitude based on said upper body movement,
- measurement by said at least one sensor of an inhaling duration and an exhaling duration of the breathing of said person based on said upper body movement,
- measurement by said at least one sensor of a current breathing rate based on said inhaling duration and said exhaling duration,
- comparison by an electronic control unit of the inhaling duration to the exhaling duration,
- comparison by said electronic control unit of the current breathing rate to a mean breathing rate, and based on said comparison, characterization by said electronic control unit of the current breathing rate as fast, average, or slow,
- computation by said electronic control unit of a maximum breathing amplitude of the breathing based a maximum breathing depth,
- comparison by said electronic control unit of said maximum breathing amplitude to a mean breathing amplitude, and based on said comparison, characterization by said electronic control unit of the breathing amplitude as flat, average, or deep,
- based on the characterization of the breathing amplitude, on the characterization of the current breathing rate and on the comparison of the inhaling duration to the exhaling duration, determination by said electronic control unit of the stress level of the person within the cabin.

In a non-limitative embodiment, said method further comprises the detection by the electronic control unit of a yawning of said person based on said upper body movement, said yawning defining the maximum breathing depth.

Some embodiments of methods and/or apparatus in accordance with embodiments of the present invention are now described, by way of example only, and with reference to the accompanying drawings, in which:
Fig. 1 is a schematic diagram which illustrates a system for defining a stress level of a person within a cabin of a vehicle, said system comprising at least one sensor and one electronic control unit, according to a non-limitative embodiment of the invention,
Fig. 2 is a diagram of a breathing of a person comprising an inhaling duration and an exhaling duration measured by the at least one sensor of the system of figure 1, according to a non-limitative embodiment,
Fig. 3 is a diagram of a yawing of a person measured by the at least one sensor of the system of figure 1, according to a non-limitative embodiment,
Fig. 4 is a schematic diagram of a method for defining a stress level of a person within a cabin of a vehicle that is carried out by the system of figure 1, according to a non-limitative embodiment.

In the following description, well-known functions or constructions by the man skilled in the art are not described in detail since they would obscure the invention in unnecessary detail.

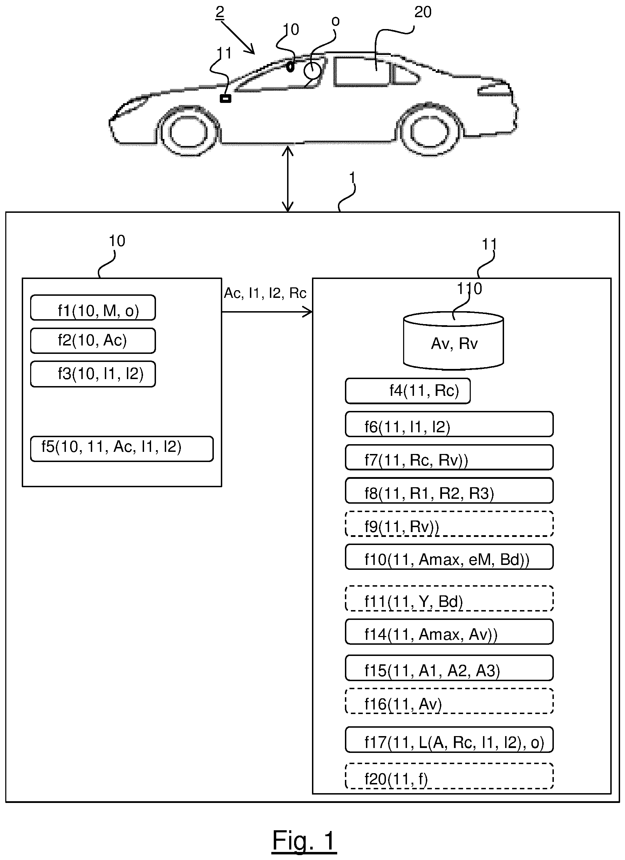

The present invention relates to a system 1 for defining a stress level L of a person o within a cabin 20 of a vehicle 2. Said system 1 is described in reference to figures 1 to 3, according to a non-limitative embodiment. In a non-limitative embodiment, said vehicle 2 is an automobile vehicle. In non-limitative examples, said automobile vehicle is a motor vehicle, or an electrical vehicle, or a hybrid vehicle.

As illustrated in figure 1, the vehicle 2 comprises a cabin 20. A person o is within said cabin 20. In the non-limitative example illustrated, the person o is the driver of said vehicle 2.

The system 1 comprises at least one sensor 10 and an electronic control unit 11. In a non-limitative embodiment, the electronic control unit 11 is part of said at least one sensor 10 or apart from said at least one sensor 10.

In non-limitative embodiments, the at least one sensor 10 is a radar sensor or an optical sensor. In a non-limitative variant of embodiment, the optical sensor is a camera. In the non-limitative illustrated example, the vehicle 2 comprises one sensor 10 that is a camera settled in the upper front inside the cabin 20.

The sensor 10 is configured to measure an upper body movement M of said person o (function illustrated in figure 1 f1(10, M, o)) and based on said upper body movement, to measure the current breathing amplitude Ac (function illustrated in figure 1 f2(10, Ac)) and the inhaling duration 11 and the exhaling duration I2 of the breathing of said person o (function illustrated in figure 1 f3(10, I1, I2)).

In a non-limitative embodiment, in order to measure the upper body movement M, the sensor 10 measures the distance between said upper body M and itself. The upper body movement is defined as either the chest, or either the belly, or both of them.

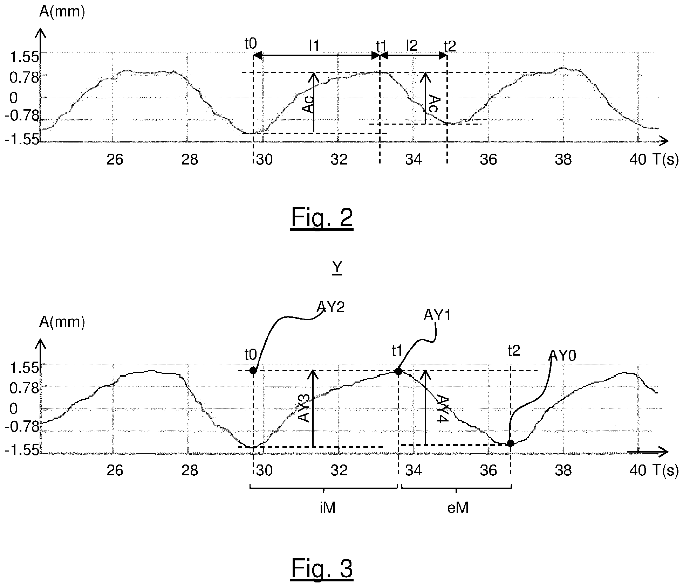

The measurement of the upper body movement M of a person o is well-known by the man skilled in the art, therefore it is not described here. The upper body movement M gives information on the breathing of the person o such as the current breathing amplitude Ac and the inhaling duration I1 and the exhaling duration I2. Figure 2 illustrates a non-limitative example of the upper body movement M of a person o. On the X-axis, one finds the time T in seconds (s), and on the Y-axis, one finds the breathing amplitude A in millimeters (mm). The inhaling duration I1 is defined as the duration between the start of inhaling t0 and the end of the inhaling t1. The end of the inhaling t1 is the defined as the closest distance from the chest and/or belly to the sensor 10. The inhaling is defined as the chest and/or belly moving towards the sensor 10.

The exhaling duration I2 is defined as the duration between the end of inhaling t1 and the end of the exhaling t2. The exhaling is defined as the chest and/or belly moving away from the sensor 10.

The measurement of the inhaling duration I1 and the exhaling duration I2 from the upper body movement M are well-known by the man skilled in the art and therefore are not described here.

The current breathing amplitude Ac can be determined between the end of the inhaling t1 and the end of the exhaling t2, or by the difference between the start of the inhaling t0 and the end of the inhaling t1 while inhaling. The current breathing amplitude Ac is the maximum chest and/or belly motion amplitude.

The electronic control unit 11 is configured to measure a current breathing rate Rc based on said inhaling duration I1 and said exhaling duration I2 (function illustrated in figure 1 f4(11, Rc)).

The current breathing rate Rc is measured based on a plurality of inhaling durations I1 and exhaling duration I2. The measurement of said current breathing rate Rc is well-known by the man skilled in the art, therefore it is not described here. In a non-limitative embodiment, the current breathing rate Rc is a long term measurement performed during one journey of said vehicle 2.

The current breathing amplitude Ac, the inhaling duration I1, the exhaling durationI2 are sent by the sensor 10 to the electronic control unit 11 (function illustrated in figure 1 f5(10, 11, Ac, I1, I2)).

With these data, the electronic control unit 11 is configured to compare the inhaling duration I1 and the exhaling durationI2 (function illustrated in figure 1 f6(11, I1, I2)) and see if they are equal or if one duration is lower than the other one.

The electronic control unit 11 is further configured to compare the current breathing rate Rc to a mean breathing rate Rv (function illustrated in figure 1 f7(11, Rc, Rv)) and based on said comparison, to characterize the breathing rate as slow R1, or average R2, or fast R3 (function illustrated in figure 1 f8(11, R1, R2, R3)). In a non-limitative embodiment, the current breathing rate Rc is :
- fast when the current breathing rate Rc is equal or superior to 1.3 times to the mean breathing rate Rv,
- average when the current breathing rate Rc is equal to the mean breathing rate Rv,
- slow when the current breathing rate Rc is inferior to 0.7 times the mean breathing rate Rv.

In a non-limitative embodiment, the mean breathing rate Rv is stored in a non-volatile memory 110 of the electronic control unit 11. In a non-limitative embodiment, the mean breathing rate Rv comes from permanently measures of the current breathing rate Rc of the person o and is derived from said measures, every time the person o uses the vehicle 2, therefore during a plurality of journeys of the vehicle 2 contrary to the current breathing rate Rc that is measured during only one journey. Therefore, in a non-limitative embodiment, the electronic control unit 11 is further configured to compute the mean breathing rate Rv (function illustrated in figure 1 f9(11, Rv)). This function being optional, it is illustrated in a dash line.

The electronic control unit ECU is further configured to compute a maximum breathing amplitude Amax based on an exhaling movement eM of a maximum breathing depth Bd (function illustrated in figure 1 f10(11, Amax, eM, Bd)).

The maximum breathing depth Bd allows the capture of the maximum breathing amplitude Amax that will be used as a reference. It will permit to know how deep the person o can breathe to really differentiate a flat breathing amplitude A from a deep breathing amplitude A. Without the maximum breathing depth Bd, it is more difficult to differentiate between the flat and deep breathing amplitudes. There could be less than only 1 millimeter difference.

In a first non-limitative embodiment, the maximum breathing depth Bd comes from a yawing Y of said person o. Hence, in a non-limitative embodiment, the electronic control unit 11 is further configured to detect a yawning Y based on said upper body movement M acquired by the sensor 10, said yawning Y defining the maximum breathing depth Bd (function illustrated in figure 1 f11(11, Y, Bd)). This function being optional, it is illustrated in a dash line. Said yawning Y is determined from said upper body movement M. The maximum breathing amplitude Amax will be the maximum chest and/or belly motion amplitude in the moment of yawning y considering the exhaling movement eM. The yawing Y allows the easily capture of the maximum breathing amplitude Amax that will be used as a reference. It avoids asking the person o to deeply inhale and exhale as the measure of the yawing Y is performed without his/her knowledge.

Figure 3 illustrates a non-limitative example of a yawning Y of a person o. On the X-axis, one finds the time T in seconds (s), and on the Y-axis, one finds the breathing amplitude A in millimeters (mm). It is to be noted that yawning is detected if once the amplitude is much higher than current or mean breathing amplitude.

At the end of the yawning Y (t2), one exhales while at the beginning of the yawning Y (t0), one inhales. On the diagram of figure 3, one observes:
- AY0 the amplitude that corresponds to the relative distance of the chest and/or belly to the sensor 10 at the end of the yawning Y (t2) (finish of exhaling),
- AY1 the amplitude that corresponds to the relative distance of the chest and/or belly to the sensor 10 at the peak of the yawning Y (t1) (finish of inhaling)
- AY2 the amplitude that corresponds to the relative distance of the chest and/or belly to the sensor 10 at the beginning of the yawning Y (t0).

The maximum breathing amplitude Amax is computed based on either the inhaling movement iM or either the exhaling movement eM or either on both inhaling movement iM and exhaling movement eM. On the diagram of figure 3, one observes:
- AY3 the amplitude that corresponds to the distance of the chest and/or belly between the start of the yawning Y (t0) and the peak of the yawning Y (t1) during the inhaling movement iM,
- AY4 the amplitude that corresponds to the distance of the chest and/or belly between the peak of the yawing Y (t1) and the end of the yawning Y (t2) during the exhaling movement eM.

So, the maximum breathing amplitude Amax is defined as either AY3, either AY4, or either the average of AY3 and AY4.

In a second non-limitative embodiment, one asks the person o through a human interface machine (not illustrated) to deeply inhale and exhale as much possible in order to have a maximum breathing depth Bd and to compute the maximum breathing amplitude Amax. Hence, the maximum breathing amplitude Amax is computed through a calibration.

The electronic control unit 11 is further configured to compare said maximum breathing amplitude Amax to a mean breathing amplitude Av (function illustrated in figure 1 f14(11, Amax, Av)) and based on said comparison, to characterize the breathing amplitude A as flat A1, or average A2, or deep A3 (function illustrated in figure 1 f15(11, A1, A2, A3)). In a non-limitative embodiment, the breathing amplitude A is flat when the mean breathing amplitude Av is between 0.3 to 0.5 times the maximum breathing amplitude Amax, and the breathing amplitude A is deep when the mean breathing amplitude Av is between 0.7 to 0.9 times the maximum breathing amplitude Amax. In a non-limitative embodiment, the breathing amplitude A is average when mean breathing amplitude Av is 0.6 times the maximum breathing amplitude Amax.

In a non-limitative embodiment, the mean breathing amplitude Av is stored in a non-volatile memory 110 of the electronic control unit 11. In a non-limitative embodiment, the mean breathing amplitude Av comes from permanently measures of the breathing amplitude A of the person o and is derived from said measures. The permanently measures of the breathing amplitude A are the current breathing amplitude Ac measured (function f1 described before). Therefore, in a non-limitative embodiment, the electronic control unit 11 is further configured to compute the mean breathing amplitude Av based on a plurality of measures of the current breathing amplitude Ac (function illustrated in figure 1 f16(11, Av)). This function being optional, it is illustrated in a dash line.

Based on the characterization of the breathing amplitude A, on the characterization of current breathing rate Rc and on the comparison of the inhaling duration I1 to the exhaling duration I2, the electronic control unit 11 is further configured to determine the stress level L of said person o within the cabin 20 of the vehicle 2 (function illustrated in figure 1 f17(11, L(A, Rc, I1, I2), o)).

In a non-limitative embodiment, the stress level L is defined as relaxed, slightly stressed and stressed.

In a non-limitative embodiment, the stress level L is relaxed when:
- the current breathing rate Rc is slow, and
- the breathing amplitude A is deep, and
- the inhaling duration I1 is shorter than to the exhaling duration I2.

In a non-limitative embodiment, the stress level L is slightly stressed when:
- the current breathing rate Rc is average, and
- the breathing amplitude A is equal to the mean breathing amplitude Av, and
- the inhaling duration I1 is equal to the exhaling duration I2.

In a non-limitative variant of embodiment, the stress level L is stressed when:
- the current breathing rate Rc is fast, and
- the breathing amplitude A is flat, and
- the inhaling duration I1 is longer than the exhaling duration I2.

In a non-limitative variant of embodiment, a stress level L is composed of different sub-levels, also called stress sub-levels, defined according in particular to different times of comparison of the current breathing rate Rc and of the breathing amplitude A. It allows a precise tuning of the different levels L. Hence, in non-limitative examples, the lowest stress level L, i.e. the relaxed one, is composed of four sub-levels L1 to L4 ; the median stressed level, i.e. the slightly stressed, is composed of two sub-levels L5 to L6, and the highest stress level L, i.e. the stressed one, is composed of only one sub-level L7.

In a non-limitative example, the sub-level L1 (relaxed) is defined when:
- the current breathing rate Rc is equal to the mean breathing rate Rv, and
- the breathing amplitude A is equal to the mean breathing amplitude Av, and
- the inhaling duration I1 is equal to the exhaling duration I2.

In a non-limitative example, the sub-level L2 (relaxed) is defined when:
- the current breathing rate Rc is equal to 1.1 times the breathing rate Rv, and
- the breathing amplitude A is equal to the mean breathing amplitude Av, and
- the inhaling duration I1 is equal to the exhaling duration I2.

In a non-limitative example, the sub-level L3 (relaxed) is defined when:
- the current breathing rate Rc is equal to 1.2 times the breathing rate Rv, and
- the breathing amplitude A is equal to the mean breathing amplitude Av, and
- the inhaling duration I1 is equal to the exhaling duration I2.

In a non-limitative example, the sub-level L4 (relaxed) is defined when:
- the current breathing rate Rc is equal to 1.1 times the breathing rate Rv, and
- the breathing amplitude A is equal to 0.9 times the mean breathing amplitude Av, and
- the inhaling duration I1 is equal to the exhaling duration I2.

In a non-limitative example, the sub-level L5 (slightly stressed) is defined when:
- the current breathing rate Rc is equal to 1.2 times the mean breathing rate Rv, and
- the breathing amplitude A is equal to 0.8 times the mean breathing amplitude Av, and
- the inhaling duration I1 is equal to the exhaling duration I2.

In a non-limitative example, the sub-level L6 (slightly stressed) is defined when:
- the current breathing rate Rc is equal to 1.5 times the mean breathing rate Rv, and
- the breathing amplitude A is equal to 0.5 times the mean breathing amplitude Av, and
- the inhaling duration I1 is lower than the exhaling duration I2.

In a non-limitative example, the sub-level L7 (stressed) is defined when:
- the current breathing rate Rc equal to 1.8 times the mean breathing rate Rv, and
- the breathing amplitude A is equal to 0.3 times the mean breathing amplitude Av, and
- the inhaling duration I1 is significantly lower than the exhaling duration I2.

Hence, based on the stress level L, the electronic control unit 11 can deduce if the person o is relaxed, slightly stressed or stressed. Then, in a non-limitative embodiment, based on the stress level L, the electronic control unit 11 can execute a security function.

The security function f can be a combination of these non-limitative examples.

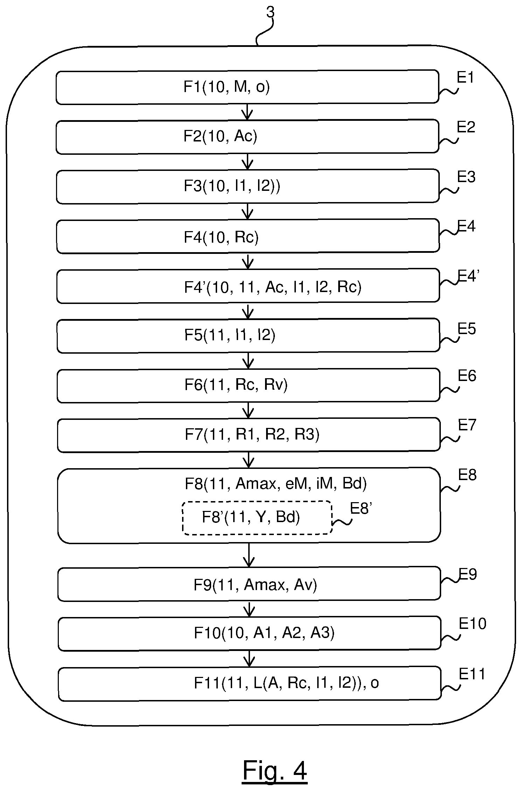

Hence the system 1 is configured to carry out a method 3 for defining a stress level L of a person o within a cabin 20 of a vehicle 2, said method 3 being described in reference to figure 4.

As illustrated in figure 4, the method 3 comprises the following steps.

In step E1 illustrated F1(10, M, o), the at least one sensor 10 measures the upper body movement M of the person o.

In step E2 illustrated F2(10, Ac), the at least one sensor 10 measures the current breathing amplitude Ac based on said upper body movement M.

In step E3 illustrated F3(10, I1, I2), the at least one sensor 10 measures the inhaling duration I1 and the exhaling duration I2 of the breathing of said person o.

It is to be noted that the steps E2 and E3 may be performed in parallel or one after another in any order.

In step E4 illustrated F4(10, Rc), the at least one sensor 10 measures the current breathing rate Rc based on said inhaling duration I1 and said exhaling duration I2.

These data (Ac, I1, I2, Rc) are transmitted by the at least one sensor 10 to the electronic control unit 11 (step E4' illustrated F4'(10, 11, Ac, I1, I2, Rc)).

In step E5 illustrated F5(11, I1, I2), the electronic control unit 11 compares the inhaling duration I1 to the exhaling duration I2.

In step E6 illustrated F6(11, Rc, Rv), the electronic control unit 11 compares the current breathing rate Rc to the mean breathing rate Rv.

In step E7 illustrated F7(11, R1, R2, R3), based on said comparison, said electronic control unit 11 characterizes the current breathing rate Rc as slow R1, or average R2, or fast R3.

In step E8 illustrated F8(11, Amax, eM, iM, Bd), the electronic control unit 11 computes the maximum breathing amplitude Amax of the breathing based on either an exhaling movement eM of a maximum breathing depth Bd, an inhaling movement iM of a maximum breathing depth Bd, either both exhaling movement eM and inhaling movement iM.

In a non-limitative embodiment, the method 3 comprises a sub-step E8' illustrated F8'(11, Y, Bd) of detecting by the electronic control unit 11 a yawning Y of the person o based on said upper body movement M, said yawning Y defining the maximum breathing depth Bd. This sub-step being optional, it is illustrated in a dash-line.

In step E9 illustrated F9(11, Amax, Av), the electronic control unit 11 compares said maximum breathing amplitude Amax to a mean breathing amplitude Av.

In step E10 illustrated F10(11, A1, A2, A3), based on said comparison, said electronic control unit 11 characterizes the breathing amplitude A as flat A1 or average A2, or deep A3.

In step E11 illustrated F11(11, L(A, Rc, I1, I2), o)), based on the characterization of the breathing amplitude A, on the characterization of current breathing rate Rc and on the comparison of the inhaling duration I1 to the exhaling duration I2, said electronic control unit 11 determines the stress level L of the person 0 within the cabin 20.

It is to be understood that the present invention is not limited to the aforementioned embodiments or applications, and variations and modifications may be made without departing from the scope of the invention. Hence, the invention may be used in other fields than the vehicle field, such as the medical field or the field of sport.

Hence, some embodiments of the invention may comprise one or a plurality of the following advantages:
- it allows to define the stress level L of a person o within the cabin 20 of a vehicle 2,
- Hence, according to the stress level defined, some security functions or vehicle assist functions can be performed,
- it is easy to implement.

## Claims

1. System (1) for defining a stress level (L) of a person (o) within a cabin (20) of a vehicle (2), wherein said system (1) comprises:
- at least one sensor (10) configured to measure:
o an upper body movement (M) of a person (o),
o a current breathing amplitude (Ac) based on said upper body movement (M),
o an inhaling duration (I1) and an exhaling duration (I2) of the breathing of said person (o) based on said upper body movement (M),
o a current breathing rate (Rc) based on said inhaling duration (I1) and said exhaling duration (I2),
- an electronic control unit (11) configured to:
o compare the inhaling duration (I1) to the exhaling duration (I2),
o compare said current breathing rate (Rc) to a mean breathing rate (Rv), and based on said comparison, characterize the current breathing rate (Rc) as slow (R1), average (R2) or fast (R3),
o compute a maximum breathing amplitude (Amax) based on a maximum breathing depth (Bd),
o compare said maximum breathing amplitude (Amax) to a mean breathing amplitude (Av), and based on said comparison, characterize the breathing amplitude (A) as flat (A1), average (A2), or deep (A3),
o based on the characterization of the breathing amplitude (A), on the characterization of the current breathing rate (Rc) and on the comparison of the inhaling duration (I1) to the exhaling duration (I2), determine the stress level (L) of said person (o) within the cabin (20).

2. System (1) according to claim 1, wherein said sensor (10) is a radar sensor or an optical sensor.

3. System (1) according to any preceding claims, wherein the electronic control unit (11) is further configured to detect a yawning (Y) based on said upper body movement (M), said yawning (Y) defining the maximum breathing depth (Bd).

4. System (1) according to any preceding claims, wherein the measurement of the current breathing rate (Rc) is a long term measurement performed during a journey of said vehicle (2).

5. System (1) according to any preceding claims, wherein the stress level (L) is defined as relaxed (L1-L4), slightly stressed (L5-6), and stressed (L7).

6. System (1) according to claim 5, wherein the stress level (L) is relaxed when:
- the current breathing rate (Rc) is slow, and
- the breathing amplitude (A) is deep, and
- the inhaling duration (I1) is shorter than the exhaling duration (I2).

7. System (1) according to claim 5, wherein the stress level (L) is slightly stressed when:
- the current breathing rate (Rc) is average, and
- the breathing amplitude (A) is equal to the mean breathing amplitude (Av), and
- the inhaling duration (I1) is equal to the exhaling duration (I2).

8. System (1) according to claim 5, wherein the stress level (L) is stressed when:
- the current breathing rate (Rc) is fast, and
- the breathing amplitude (A) is flat, and
- the inhaling duration (I1) is longer than the exhaling duration (I2).

9. System (1) according to any preceding claims, wherein the stress level (L) comprises a plurality of sub-levels (L1-L7).

10. System (1) according to any preceding claims, wherein the mean breathing amplitude (Av) is computed from a plurality of measures of the current breathing amplitude (Ac).

11. Method (3) for defining a stress level (L) of a person (o) within a cabin (20) of a vehicle (2), wherein said method (3) comprising:
- measurement (E1) by at least one sensor (10) of an upper body movement (M) of a person (o),
- measurement (E2) by said at least one sensor (10) of a current breathing amplitude (Ac) based on said upper body movement (M),
- measurement (E3) by said at least one sensor (10) of an inhaling duration (I1) and an exhaling duration (I2) of the breathing of said person (o) based on said upper body movement (M),
- measurement (E4) by said at least one sensor (10) of a current breathing rate (Rc) based on said inhaling duration (I1) and said exhaling duration (I2),
- comparison (E5) by an electronic control unit (11) of the inhaling duration (I1) to the exhaling duration (I2),
- comparison (E6) by said electronic control unit (11) of the current breathing rate (Rc) to a mean breathing rate (Rv), and based on said comparison, characterization (E7) by said electronic control unit (11) of the current breathing rate (Rc) as fast, average, or slow,
- computation (E8) by said electronic control unit (11) of a maximum breathing amplitude (Amax) of the breathing based a maximum breathing depth (Bd),
- comparison (E9) by said electronic control unit (11) of said maximum breathing amplitude (Amax) to a mean breathing amplitude (Av), and based on said comparison, characterization (E10) by said electronic control unit (11) of the breathing amplitude (A) as flat, average, or deep,
- based on the characterization of the breathing amplitude (A), on the characterization of the current breathing rate (Rc) and on the comparison of the inhaling duration (I1) to the exhaling duration (I2), determination (E11) by said electronic control unit (11) of the stress level (L) of the person (o) within the cabin (20).

12. Method (3) according to the preceding claim, wherein said method (3) further comprises the detection (E8') by the electronic control unit (11) of a yawning (Y) of said person (o) based on said upper body movement (M), said yawning (Y) defining the maximum breathing depth (Bd).

## Patentansprüche

1. System (1) zum Definieren eines Stressniveaus (L) einer Person (o) innerhalb eines Fahrgastraums (20) eines Fahrzeugs (2), wobei das System (1) umfasst:
- mindestens einen Sensor (10), der so konfiguriert ist, dass er Folgendes misst:
o eine Oberkörperbewegung (M) einer Person (o),
o eine aktuelle Atemamplitude (Ac) basierend auf der Oberkörperbewegung (M),
o eine Einatmungsdauer (11) und eine Ausatmungsdauer (12) des Atmens der Person (o) basierend auf der Oberkörperbewegung (M),
o eine aktuelle Atemfrequenz (Rc) basierend auf der Einatmungsdauer (11) und der Ausatmungsdauer (12),
- eine elektronische Steuereinheit (11), die konfiguriert ist zum:
o Vergleichen der Einatmungsdauer (11) mit der Ausatmungsdauer (12),
o Vergleichen der aktuellen Atemfrequenz (Rc) mit einer mittleren Atemfrequenz (Rv) und Charakterisieren der aktuellen Atemfrequenz (Rc) basierend auf dem Vergleich als langsam (R1), durchschnittlich (R2) oder schnell (R3),
o Berechnen einer maximalen Atemamplitude (Amax) basierend auf einer maximalen Atemtiefe (Bd),
o Vergleichen der maximalen Atemamplitude (Amax) mit einer mittleren Atemamplitude (Av) und Charakterisieren der aktuellen Atemamplitude (A) basierend auf dem Vergleich als flach (A1), durchschnittlich (A2) oder tief (A3),
o Bestimmen des Stressniveaus (L) der Person (o) innerhalb des Fahrgastraums (20) basierend auf der Charakterisierung der Atemamplitude (A), auf der Charakterisierung der aktuellen Atemfrequenz (Rc) und auf dem Vergleich der Einatmungsdauer (I1) mit der Ausatmungsdauer (12).

2. System (1) nach Anspruch 1, wobei der Sensor (10) ein Radarsensor oder ein optischer Sensor ist.

3. System (1) nach einem der vorhergehenden Ansprüche, wobei die elektronische Steuereinheit (11) ferner zum Erkennen eines Gähnens (Y) basierend auf der Oberkörperbewegung (M) konfiguriert ist, wobei das Gähnen die maximale Atemtiefe (Bd) definiert.

4. System (1) nach einem der vorhergehenden Ansprüche, wobei die Messung der aktuellen Herzfrequenz (Rc) eine Langzeitmessung ist, die während einer Fahrt des Fahrzeugs (2) durchgeführt wird.

5. System (1) nach einem der vorhergehenden Ansprüche, wobei das Stressniveau (L) als entspannt (L1-L4), leicht gestresst (L5-6) und gestresst (L7) definiert ist.

6. System (1) nach Anspruch 5, wobei das Stressniveau (L) entspannt ist, wenn:
- die aktuelle Atemfrequenz (Rc) langsam ist, und
- die Atemamplitude (A) tief ist, und
- die Einatmungsdauer (I1) kürzer als die Ausatmungsdauer (12) ist.

7. System (1) nach Anspruch 5, wobei das Stressniveau (L) leicht gestresst ist, wenn:
- die aktuelle Atemfrequenz (Rc) durchschnittlich ist, und
- die Atemamplitude (A) gleich der mittleren Atemamplitude (Av) ist, und
- die Einatmungsdauer (11) gleich der Ausatmungsdauer (12) ist.

8. System (1) nach Anspruch 5, wobei das Stressniveau (L) gestresst ist, wenn:
- die aktuelle Atemfrequenz (Rc) schnell ist, und
- die Atemamplitude (A) flach ist, und
- die Einatmungsdauer (I1) länger als die Ausatmungsdauer (12) ist.

9. System (1) nach einem der vorhergehenden Ansprüche, wobei das Stressniveau (L) eine Mehrzahl von Teilniveaus (L1-L7) umfasst.

10. System (1) nach einem der vorhergehenden Ansprüche, wobei die mittlere Atemamplitude (Av) aus einer Mehrzahl von Maßen der aktuellen Atemamplitude (Ac) berechnet wird.

11. Verfahren (3) zum Definieren eines Stressniveaus (L) einer Person (o) innerhalb eines Fahrgastraums (20) eines Fahrzeugs (2), wobei das Verfahren (3) umfasst:
- Messen (E1) einer Oberkörperbewegung (M) einer Person (o) durch mindestens einen Sensor (10),
- Messen (E2) einer aktuellen Atemamplitude (Ac) durch den mindestens einen Sensor (10) basierend auf der Oberkörperbewegung (M),
- Messen (E3) einer Einatmungsdauer (11) und einer Ausatmungsdauer (12) des Atmens der Person (o) durch den mindestens einen Sensor (10) basierend auf der Oberkörperbewegung (M),
- Messen (E4) einer aktuellen Atemfrequenz (Rc) durch den mindestens einen Sensor (10) basierend auf der Einatmungsdauer (11) und der Ausatmungsdauer (12),
- Vergleichen (E5) der Einatmungsdauer (11) durch eine elektronische Steuereinheit (11) mit der Ausatmungsdauer (12),
- Vergleichen (E6) der aktuellen Atemfrequenz (Rc) durch die elektronische Steuereinheit (11) mit einer mittleren Atemfrequenz (Rv) und Charakterisieren (E7) der aktuellen Atemfrequenz (Rc) durch die elektronische Steuereinheit (11) als schnell, durchschnittlich oder langsam,
- Berechnen (E8) einer maximalen Atemamplitude (Amax) des Atmens durch die elektronische Steuereinheit (11) basierend auf einer maximalen Atemtiefe (Bd),
- Vergleichen (E9) der maximalen Atemamplitude (Amax) durch die elektronische Steuereinheit (11) mit einer mittleren Atemamplitude (Av) und Charakterisieren (E10) der Atemamplitude (A) durch die elektronische Steuereinheit (11) als flach, durchschnittlich oder tief,
- Bestimmen (E11) des Stressniveaus (L) der Person (o) innerhalb des Fahrgastraums (20) durch die elektronische Steuereinheit (11) basierend auf der Charakterisierung der Atemamplitude (A), auf der Charakterisierung der aktuellen Atemfrequenz (Rc) und auf dem Vergleich der Einatmungsdauer (11) mit der Ausatmungsdauer (12).

12. Verfahren (3) nach einem der vorhergehenden Ansprüche, wobei das Verfahren (3) ferner das Erkennen (E8') eines Gähnens (Y) der Person (o) durch die elektronischen Steuereinheit (11) basierend auf der Oberkörperbewegung (M) umfasst, wobei das Gähnen (Y) die maximale Atemtiefe (Bd) definiert.

## Revendications

1. Système (1) de définition d'un niveau de stress (L) d'une personne (o) au sein d'un habitacle (20) d'un véhicule (2), dans lequel ledit système (1) comprend :
- au moins un capteur (10) configuré pour mesurer :
o un mouvement du haut du corps (M) d'une personne (o),
o une amplitude respiratoire courante (Ac) basée sur ledit mouvement du haut du corps (M),
o une durée d'inspiration (I1) et une durée d'expiration (12) de la respiration de ladite personne (o) en fonction dudit mouvement du haut du corps (M),
o une fréquence respiratoire courante (Rc) basée sur ladite durée d'inspiration (I1) et ladite durée d'expiration (12),
- une unité de commande électronique (11) configurée pour :
o comparer la durée d'inspiration (I1) à la durée d'expiration (12),
o comparer ladite fréquence respiratoire courante (Rc) à une fréquence respiratoire moyenne (Rv) et, sur la base de cette comparaison, caractériser la fréquence respiratoire courante (Rc) comme lente (R1), moyenne (R2) ou rapide (R3),
o calculer une amplitude respiratoire maximale (Amax) sur la base d'une profondeur respiratoire maximale (Bd),
o comparer ladite amplitude respiratoire maximale (Amax) à une amplitude respiratoire moyenne (Av) et, sur la base de cette comparaison, caractériser l'amplitude respiratoire (A) comme faible (A1), moyenne (A2) ou profonde (A3),
o sur la base de la caractérisation de l'amplitude respiratoire (A), de la caractérisation de la fréquence respiratoire courante (Rc) et de la comparaison de la durée d'inspiration (I1) à la durée d'expiration (12), déterminer le niveau de stress (L) de ladite personne (o) à l'intérieur de l'habitacle (20).

2. Système (1) selon la revendication 1, dans lequel ledit capteur (10) est un capteur radar ou un capteur optique.

3. Système (1) selon l'une quelconque des revendications précédentes, dans lequel l'unité de commande électronique (11) est en outre configurée pour détecter un bâillement (Y) sur la base dudit mouvement du haut du corps (M), ledit bâillement (Y) définissant la profondeur maximale de respiration (Bd).

4. Système (1) selon l'une quelconque des revendications précédentes, dans lequel la mesure de la fréquence respiratoire courante (Rc) est une mesure à long terme réalisée au cours d'un trajet dudit véhicule (2) .

5. Système (1) selon l'une quelconque des revendications précédentes, dans lequel le niveau de stress (L) est défini par : détendu (L1-L4), légèrement stressé (L5-6) et stressé (L7).

6. Système (1) selon la revendication 5, dans lequel le niveau de stress (L) est qualifié de détendu lorsque :
- la fréquence respiratoire courante (Rc) est lente, et
- l'amplitude respiratoire (A) est profonde, et
- la durée d'inspiration (I1) est plus courte que la durée d'expiration (12).

7. Système (1) selon la revendication 5, dans lequel le niveau de stress (L) est qualifié de légèrement stressé lorsque :
- la fréquence respiratoire courante (Rc) est moyenne, et
- l'amplitude respiratoire (A) est égale à l'amplitude respiratoire moyenne (Av), et
- la durée d'inspiration (I1) est égale à la durée d'expiration (12).

8. Système (1) selon la revendication 5, dans lequel le niveau de stress (L) est qualifié de stressé lorsque :
- la fréquence respiratoire courante (Rc) est rapide, et
- l'amplitude respiratoire (A) est faible, et
- la durée d'inspiration (I1) est plus longue que la durée d'expiration (12).

9. Système (1) selon l'une quelconque des revendications précédentes, dans lequel le niveau de stress (L) comprend une pluralité de sous-niveaux (L1-L7).

10. Système (1) selon l'une quelconque des revendications précédentes, dans lequel l'amplitude respiratoire moyenne (Av) est calculée à partir d'une pluralité de mesures de l'amplitude respiratoire courante (Ac) .

11. Procédé (3) de définition d'un niveau de stress (L) d'une personne (o) au sein d'un habitacle (20) d'un véhicule (2), dans lequel ledit procédé (3) comprend :
- la mesure (E1), par au moins un capteur (10), d'un mouvement du haut du corps (M) d'une personne (o),
- la mesure (E2), par ledit au moins un capteur (10), d'une amplitude respiratoire courante (Ac) sur la base dudit mouvement du haut du corps (M),
- la mesure (E3), par ledit au moins un capteur (10), d'une durée d'inspiration (I1) et d'une durée d'expiration (12) de la respiration de ladite personne (o) sur la base dudit mouvement du haut du corps (M),
- la mesure (E4), par ledit au moins un capteur (10), d'une fréquence respiratoire courante (Rc) sur la base de ladite durée d'inspiration (I1) et de ladite durée d'expiration (12),
- la comparaison (E5), par une unité de commande électronique (11), de la durée d'inspiration (I1) à la durée d'expiration (12),
- la comparaison (E6), par ladite unité de commande électronique (11), de la fréquence respiratoire courante (Rc) à une fréquence respiratoire moyenne (Rv), et sur la base de ladite comparaison, la caractérisation (E7), par ladite unité de commande électronique (11), de la fréquence respiratoire courante (Rc) comme étant rapide, moyenne ou lente,
- le calcul (E8), par ladite unité de commande électronique (11), d'une amplitude respiratoire maximale (Amax) de la respiration sur la base d'une profondeur respiratoire maximale (Bd),
- la comparaison (E9), par ladite unité de commande électronique (11), de ladite amplitude respiratoire maximale (Amax) à une amplitude respiratoire moyenne (Av) et, sur la base de ladite comparaison, la caractérisation (E10), par ladite unité de commande électronique (11), de l'amplitude respiratoire (A) comme étant faible, moyenne ou profonde,
- sur la base de la caractérisation de l'amplitude respiratoire (A), de la caractérisation de la fréquence respiratoire courante (Rc) et de la comparaison de la durée d'inspiration (I1) à la durée d'expiration (12), la détermination (E11), par ladite unité de commande électronique (11), du niveau de stress (L) de la personne (o) à l'intérieur de l'habitacle (20).

12. Procédé (3) selon la revendication précédente, dans lequel ledit procédé (3) comprend en outre de détecter (E8') par l'unité électronique de commande (11) un bâillement (Y) de ladite personne (o) sur la base dudit mouvement du haut du corps (M), ledit bâillement (Y) définissant la profondeur respiratoire maximale (Bd).
